# EUROPÄISCHE PATENTSCHRIFT

(11) **EP 2 931 210 B1**
(45) Veröffentlichungstag und Bekanntmachung des Hinweises auf die Patenterteilung: **20.06.2018**
(21) Anmeldenummer: 13798675.8
(22) Anmeldetag: 02.12.2013
(51) Int. Cl.: A61K 8/06, A61K 8/39, A61K 8/898, A61K 8/20, A61K 8/31, A61K 8/33, A61K 8/37, A61Q 19/00

(54) **QUICK BREAKING EMULSION**
QUICK-BREAKING EMULSION
ÉMULSION À EFFET "QUICK BREAK"

(30) Priorität: 11.12.2012 DE 102012222797
(43) Veröffentlichungstag der Anmeldung: 21.10.2015
(73) Patentinhaber: Beiersdorf AG, 20253 Hamburg (DE)
(72) Erfinder: RATSCHOW, Cecile, 22761 Hamburg (DE); MEYER, Christiane, 20357 Hamburg (DE); HAGENS, Ralf, 25479 Ellerau (DE); SCHEEDE, Stefan, 22529 Hamburg (DE)
(74) Vertreter: Hartmann, Jost
(86) Internationale Anmeldenummer: PCT/EP2013/075224
(87) Internationale Veröffentlichungsnummer: WO 2014/090615

(56) Entgegenhaltungen:
- EP-A2- 2 319 484
- WO-A2-2013/120829

## Beschreibung

Die Erfindung umfasst kosmetische oder dermatologische Emulsionszubereitungen, die bei Applikation auf der Haut aufbrechen und sichtbare Wassertropfen freisetzen (Quick Breaking Emulsions). Die Emulsionen sind sensorisch attraktiv, wirken kühlend und pflegend.

Als kosmetische oder medizinische Zubereitungen sind oftmals Emulsionen, insbesondere W/O-, O/W-, O/W/O oder W/O/W-Emulsionen, im Einsatz. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten (W/O) in Form feinster Tröpfchen in der anderen Flüssigkeit dispergiert. Die Flüssigkeiten (rein oder als Lösungen) liegen in einer Emulsion in einer mehr oder weniger feinen Verteilung vor, die im Allgemeinen nur begrenzt stabil ist.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter, zum Beispiel elektrische Leitfähigkeit, Sensorik, Anfärbbarkeit der kontinuierlichen Phase, einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Der Stand der Technik kennt mehrere wesentliche Faktoren, die einen positiven Einfluss auf die Stabilität und Rheologie von Emulsionen haben.

Emulsionen benötigen zu ihrer Bildung und zur Stabilisierung im Allgemeinen einen oder mehrere Emulgatoren, Verdicker und/oder Konsistenzgeber, um über einen kosmetisch akzeptablen Zeitraum stabil zu sein, im Allgemeinen ein Jahr nach dem Öffnen einer kosmetischen Zubereitung.

Fließfähige Emulsionen werden aufgrund ihrer ansprechenden Verteilbarkeit vom Verbraucher sehr geschätzt, jedoch ist die stabile Formulierung eine technologische Herausforderung.

Zur Stabilisierung von Emulsionen werden häufig ethoxilierte Emulgatoren verwendet, die, allgemein bekannt, zu belastbaren, stabilen Emulsionszubereitungen führen und oft einen relativ breiten sensorischen Bereich abdecken können. Bekannt ist jedoch, dass ethoxilierte Emulgatoren durch ihre PEG-Einheiten als Penetrationsenhancer wirken können.

So offenbart EP 1 192 935 A2 W/O-Emulsionen umfassend Polyether wie PEG-45 (dodecyl glycol) copolymer und PEG-22 (dodecyl glycol) copolymere.

Die Verwendung von Polyethylenglykolen und/oder Polyethylenglykolderivaten und deren Abkömmlingen werden in der Öffentlichkeit kontrovers diskutiert, da sie im Verdacht stehen nach topische Applikation die Haut durchlässiger für z.B. Schadstoffe zu machen.

Weiterhin können unter Einwirkung von Sonnenstrahlung die photoinstabilen Polyethylenglykolhaltigen (PEG) - Emulgatoren zersetzt werden und Hautreaktionen auslösen.

Aus den genannten Gründen werden vom Verbraucher zunehmend kosmetische Formulierungen gesucht, die frei von dieser Substanzklasse sind.

Auch um W/O-Emulsionen sensorisch ansprechend entwickeln zu können, werden derzeit meist PEG-Emulgatoren verwendet. Da viele Verbraucher Produkte suchen, die frei von dieser Substanzklasse sind, ist es eine technologische Herausforderung W/O-Emulsionen ohne diese Stoffe zu stabilisieren und gleichzeitig ein ansprechendes Hautgefühl zu erlangen. Hinsichtlich der Stabilität ist die Herausforderung insbesondere das Scaling-up und beim Hautgefühl sind Parameter wie "schnelles und leichtes Einziehen" und "geringe Klebrigkeit" schwierig ohne PEG-Stabilisatoren zu erreichen.

Wünschenswert ist es daher, Emulsionszubereitung ohne ethoxilierte Emulgatoren zur Verfügung zu stellen, die aber dennoch möglichst breit variierbare und vor allem stabile Emulsionen darstellen.

Weiterhin müssen kosmetische oder dermatologische Zubereitungen einigen ästhetischen und sensorischen Gesichtspunkten genügen um eine ausreichende Verbraucherakzeptanz zu erlangen.

Stabile Emulsionen sind solche, die über einen längeren Zeitraum sowie bei Temperaturwechsel keinerlei Phasentrennung, Viskositätsänderungen, Koaleszenz, Ostwald-Reifung und/oder Aufrahmungen zeigen.

Eine Phasentrennung der Emulsion zwischen Lipidphase und Wasserphase ist daher während der Lagerung und Anwendung eigentlich nicht erwünscht.

Schnellbrechende Wasser-in-Öl Emulsionen (Quick Breaking Emulsions) sind bekannt. Diese Emulsionen mit einem hohen Wassergehalt brechen durch die bei der Anwendung auftretenden Scherkräfte sehr schnell und setzen dabei Wassertröpfchen frei, die mitunter sichtbar sind.

In der Literatur, z.B. in COSSMA 08/2006 als auch in happi 09/2006, werden solche schnell brechende Emulsionen auf der Basis des Emulgators Cetyl PEG/PPG-10/1 Dimethicone (Abil EM 90, Degussa) beschrieben.

W/O-Emulsionen mit hohem Wassergehalt sind als "high internal phase" Emulsionen (HIPE) beispielsweise in EP 1094889 A1 oder EP 1055424 A2 beschrieben. Hierin werden Cetyl Dimethicone Copolyol und Laurylmethicone Copolyol als W/Si-Emulgatoren offenbart.

In der DE 10 2009 022 445 A1 ist eine Sonnencreme mit SilCare® Silicone WSI (Amodimethicone Glycerocarbamat) offenbart.

In der DE 10 2011 013 342 A1 ist eine W/O Pflege Hautmilch mit Amodimethicone Glycerocarbamat beschrieben.

In der EP 2 319 484 A2 sind leicht brechende W/O-Emulsionen auf der Basis von Diisostearoyl Polyglyceryl-3 Dimer Dilinoleat und hydrophobisiertem Silika beschirieben.

Wünschenswert ist es, stabile Wasser-in-Öl Emulsionen für kosmetische oder dermatologische Zubereitungen zur Verfügung zu stellen, die ohne PEG-haltige Zusätze formuliert werden und bei Anwendung auf der Haut oder Haar schnell brechen und sichtbare Wassertropfen freisetzen.

Die Erfindung ist eine kosmetische oder dermatologische Zubereitung auf Basis einer Wasser-in-Öl Emulsion umfassend mindestens 0,1 Gew.%, bezogen auf die Gesamtmasse der Zubereitung, eines oder mehrerer W/O-Emulgatoren, eines oder mehrerer Elektrolyte und eines oder mehrerer Lipide. Die Zubereitungen umfassen Magnesiumstearat und sind frei von Polyethylenglykolen und/oder Polyethylenglykolderivaten. Die W/O-Emulgatoren werden ausgewählt aus der Gruppe Amodimethicon Glycerocarbamat und/oder Diisostearoyl Polyglyceryl-3 Dimer Dilinoleat.

Die erfindungsgemäßen Zubereitungen basieren auf einer Wasser-in-Öl Emulsion. Unter dem Begriff Wasser-in-Öl Emulsion sind dabei auch Wasser-in-Silikonöl (W-Si) Emulsionen subsummiert.

Die Zubereitung umfasst bevorzugt nur einen W/O- bzw. W/Si-Emulgator. Dieser wird gewählt aus Diisostearoyl Polyglyceryl-3 Dimer Dilinoleat (W/O) oder Amodimethicon Glycerocarbamat (W/Si). Diisostearoyl Polyglyceryl-3 Dimer Dilinoleat ist beispielsweise als Isolan PDI der Fa. Evonik erhältlich.

Amodimethicone Glycerocarbamat ist als Silcare Silicone WSi der Fa. Clariant erhältlich.

Der Anteil an einem oder beiden W/O Emulgatoren liegt im Bereich von 0,5 bis 2 Gew.%, bezogen auf die Gesamtmasse der Zubereitung.

Der Anteil an Diisostearoyl Polyglyceryl-3 Dimer Dilinoleate und/oder Amodimethicone Glycerocarbamat wird bevorzugt gewählt bis 1,5 Gew.%, insbesondere im Bereich von 0,5 Gew.% bis 1,5 Ge.w%, bezogen auf die Gesamtmasse der Zubereitung.

Vorteilhaft sind keine weiteren Emulgatoren in der Zubereitung enthalten, der Anteil an weiteren Emulgatoren ist demnach unterhalb 0,01 Gew.%, insbesondre 0 Gew.%, bezogen auf die Gesamtmasse der Zubereitung, zu wählen.

Überraschender Weise ist es durch den Einsatz dieser W/Si- und/oder W/O-Emulgatoren gelungen, die genannten technischen Herausforderung zu lösen.

Es sind erfindungsgemäß bevorzugt keine weiteren W/O-Emulgatoren zur Stabilisierung notwendig. Hydrophile Stabilisatoren wie Verdicker und Fettalkohole können jedoch erfindungsgemäß optional enthalten sein.

In gleicher Weise kann erfindungsgemäß auf den Zusatz an Polyethylenglykolen und/oder Polyethylenglykolderivaten verzichtet werden. Der Anteil an PEGs liegt daher unter 1 Gew.%, bezogen auf die Gesamtmasse der Zubereitung, um als erfindungsgemäß PEG-frei zu gelten. Bevorzugt liegt der Anteil an PEGs bei 0 Gew.%.

Im Rahmen der vorliegenden Offenbarung wird als Oberbegriff für Fette, Öle, Wachse und dergleichen der Ausdruck "Lipide" verwendet. Auch werden die Begriffe "Ölphase" und "Lipidphase" synonym angewandt.

Die erfindungsgemäßen Zubereitungen umfassen vorteilhaft mindestens 1 bis 20 Gew.%, bevorzugt 5 bis 15 Gew.%, Lipide, bezogen auf die Gesamtmasse der Zubereitung.

Der Lipid- oder Ölphase werden die Emulgatoren nicht hinzugerechnet.

Als Lipide werden bevorzugt eine oder mehrere Verbindungen aus der Gruppe der Dicaprylylether, Dibutyladipat, Isopropylstearat, Octylcocoat, Isopropylpalmitat, Caprylsäure/Caprinsäuretriglycerid, Octyldodecanol einzeln oder als Kombinationen gewählt. Bevorzugt werden Dicaprylylether, Isopropylstearat, Isoproprylpalmitat und/oder Octylcocoat gewählt, da sich in Anwendurigsstudien bei diesen Lipiden die angenehmste Sensorik bei der Anwendung eingestellt hat.

Elektrolyte sind chemische Verbindungen, die im festen, flüssigen oder gelösten Zustand in Ionen dissoziiert, die sich unter dem Einfluss eines elektrischen Feldes gerichtet bewegen können. Als Elektrolyt wird zwingend Magnesiumstearat und bevorzugt zusätzlich Natriumchlorid in den Zubereitungen eingesetzt.

Die Anteile an einem oder mehreren Elektrolyten wird bevorzugt gewählt im Bereich von 0,1 bis 4 Gew.%, bevorzugt 0,3 bis 3 Gew.%, bezogen auf die Gesamtmasse der Zubereitung.

Bei allen Anteilsangaben, die sich auf eine Gruppe von Stoffen bezieht, wie Emulgatoren, Lipide oder Elektrolyte, gelten erfindungsgemäß auch die offenbarten Anteile für die jeweiligen Einzelsubstanzen.

Konservierungsmitteln können vorteilhaft in der Zubereitung enthalten sein.

Bevorzugt sind als Konservierungsmittel Methylparaben, Phenoxyethanol, Methylisothiazolinone, Piroctone Olamine, Benzethonium Chloride und/oder Ethanol zu wählen. Bevorzugt sind folgende Mischungen zu wählen:
0,15% Methylparaben und 0,3% Phenoxyethanol
0,09% Methylisothiazolinone und 0,6% Phenoxyethanol
0,05% Piroctone Olamine und 0,6% Phenoxyethanol
0,05% Benzethonium Chloride und 0,5% Phenoxyethanol
oder 2% Ethanol und 0,6% Phenoxyethanol.

Alle Anteilsangaben beziehen sich dabei auf Gewichtsanteile bezogen auf die Gesamtmasse der Zubereitung.

Bei der Applikation, insbesondere topischen Applikation, der erfindungsgemäßen Zubereitungen werden erstaunliche Effekte beobachtet.

Die erfindungsgemäßen W/O- bzw. W/Si-Emulsionen brechen bei der Applikation und/oder dem Verreiben auf der Haut bzw. dem Haar auf und setzen sichtbare Wassertropfen frei. Der Rückstand auf der Haut nach Produktapplikation ist angenehm weich, geschmeidig und die Haut fühlt sich gepflegt an.

In Vergleichsversuchen zeigte sich, dass die erfindungsgemäßen Quick Breaking Emulsionen zudem stabil sind.

**Rezepturvergleich 1**

| Inci | Rezeptur A | Rezeptur B |
|---|---|---|
| Polysilicone-25 | 0 | 1,5 |
| Amodimethicone Glycerocarbamate | 1,5 | 0 |
| Glycerin + Glyceryl Glucoside | 0,1 | 0,1 |
| C13-16 Isoparaffin | 5 | 5 |
| Cyclomethicone | 4 | 4 |
| Parfum | 0,2 | 0,2 |
| Glycerin | 10 | 10 |
| Methylisothiazolinone | 0,09 | 0,09 |
| Phenoxyethanol | 0,6 | 0,6 |
| Magnesium Stearate | 0,3 | 0,3 |
| Sodium Chloride | 1 | 1 |
| Aqua | 77,21 | 77,21 |
| | | |
| **Stabilität** | **stabil¹** | **instabil²** |

| | | |
|---|---|---|
| ¹stabil bei Wechselschrank (-10°C/+40°C) für mindestens 7 Tage, -10°C-Lagerung für 30 Tage und bei Raumtemperatur und 40°C-Lagerung für mindestens 6 Monate. ²Phasentrennung nach 7 Tage Lagerung im Wechselschrank (-10°C/+40°C) | | |

**Rezepturvergleich 2**

| Inci | Rezeptur C | Rezeptur D |
|---|---|---|
| Amodimethicone Glycerocarbamate | 0,8 | 0 |
| Polysilicone-25 | 0 | 0,8 |
| Glycerin + Glyceryl Glucoside | 0,1 | 0,1 |
| Dicaprylyl Ether | 5 | 5 |
| Ethylhexyl Cocoate | 5 | 5 |
| Parfum | 0,2 | 0,2 |
| Glycerin | 10 | 10 |
| Methylisothiazolinone | 0,09 | 0,09 |
| Phenoxyethanol | 0,6 | 0,6 |
| Magnesium Stearate | 0,3 | 0,3 |
| Sodium Chloride | 1 | 1 |
| Aqua | 76,91 | 76,91 |
| | | |
| **Stabilität** | **stabil¹** | **instabil³** |

| | | |
|---|---|---|
| ¹stabil bei Wechselschrank (-10°C/+40°C) für mindestens 7 Tage, -10°C-Lagerung für 30 Tage und bei Raumtemperatur und 40°C-Lagerung für mindestens 6 Monate. ³Phasentrennung nach 120 Tage Lagerung bei 40°C | | |

Selbst bei höheren Mengen an Öl, die normalerweise in W/Si-Emulsionen stabiler zu formulieren sind, ist die Formel mit Polysilicone-25 instabiler als die Formel mit Amodimethicone Glycerocarbamat.

**Rezepturvergleich 3**

| Inci | Rezeptur E | Rezeptur F |
|---|---|---|
| Amodimethicone Glycerocarbamate | 1,5 | 0 |
| Polysilicone-25 | 0 | 1,5 |
| Glycerin + Glyceryl Glucoside | 0,1 | 0,1 |
| Dicaprylyl Ether | 3,5 | 5 |
| Ethylhexyl Cocoate | 3,5 | 5 |
| Parfum | 0,2 | 0,2 |
| Glycerin | 10 | 10 |
| Methylisothiazolinone | 0,09 | 0,09 |
| Phenoxyethanol | 0,6 | 0,6 |
| Magnesium Stearate | 0,3 | 0,3 |
| Sodium Chloride | 1 | 1 |
| Aqua | 79,21 | 76,21 |
| | | |
| **Stabilität** | **stabil¹** | **instabil⁴** |

| | | |
|---|---|---|
| ¹stabil bei Wechselschrank (-10°C/+40°C) für mindestens 7 Tage, -10°C-Lagerung für 30 Tage und bei Raumtemperatur und 40°C-Lagerung für mindestens 6 Monate. ⁴Phasentrennung nach 30 Tage Lagerung bei -10°C. | | |

**Rezepturvergleich 4**

| inci | Rezeptur E | Rezeptur G | Rezeptur F |
|---|---|---|---|
| Amodimethicone Glycerocarbamate | 1,5 | 0 | 0 |
| Diisostearoyl Polyglyceryl-3 Dimer Dilinoleate | 0 | 1,5 | 0 |
| Polysilicone-25 | 0 | 0 | 1,5 |
| Glycerin + Glyceryl Glucoside | 0,1 | 0,1 | 0,1 |
| Dicaprylyl Ether | 3,5 | 5 | 5 |
| Ethylhexyl Cocoate | 3,5 | 5 | 5 |
| Parfum | 0,2 | 0,2 | 0,2 |
| Glycerin | 10 | 10 | 10 |
| Methylisothiazolinone | 0,09 | 0,09 | 0,09 |
| Phenoxyethanol | 0,6 | 0,6 | 0,6 |
| Magnesium Stearate | 0,3 | 0,3 | 0,3 |
| Sodium Chloride | 1 | 1 | 1 |
| Aqua | 79,21 | 76,21 | 76,21 |
| | | | |
| **Stabilität** | **stabil¹** | **stabil¹** | **instabil⁴** |

| | | | |
|---|---|---|---|
| ¹stabil bei Wechselschrank (-10°C/+40°C) für mindestens 7 Tage, -10°C-Lagerung für 30 Tage und bei Raumtemperatur und 40°C-Lagerung für mindestens 6 Monate. ⁴Phasentrennung nach 30 Tage Lagerung bei -10°C. | | | |

Die Vergleichsuntersuchungen zeigen, dass erfindungsgemäße Zubereitungen mit den ausgewählten Emulgatoren Amodimethicone Glycerocarbamat oder Diisostearoyl Polyglyceryl-3 Dimer Dilinoleat stabile Emulsionen darstellen.

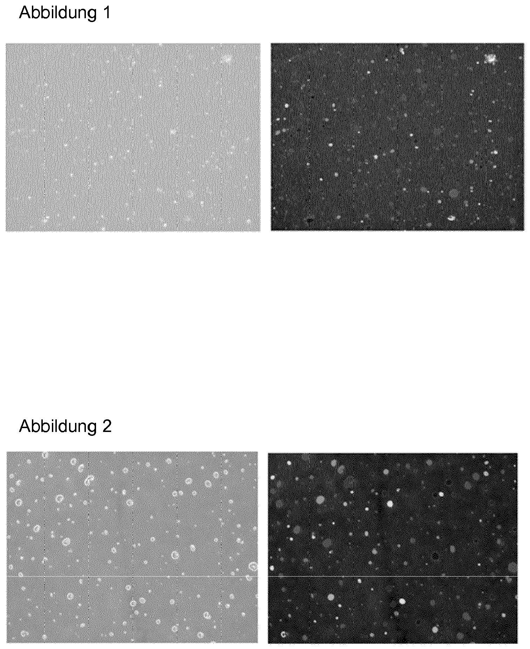

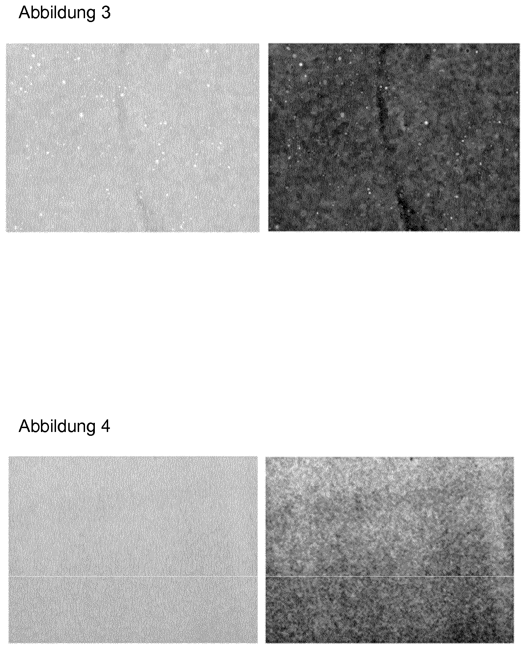

Die erfindungsgemäßen Zubereitungen können zur Freisetzung von Wassertropfen aus der Zubereitung auf der Haut verwendet werden und stellen somit schnell brechende Emulsionen dar. Dabei zeigen die freigesetzten Wassertropfen eine Größe von 500 bis 20.000 µm², bevorzugt zwischen 600 und 16.000 µm² ermittelt mit Bildanalyse KS 400 (AxioCam Cc1) auf PMMA-Plättchen. Die Anzahl der Wassertropfen beträgt mindestens 3 Tropfen/mm², insbesondere 4 bis 9 Tropfchen/mm². In den Abbildungen 1 bis 4 sind die freigesetzten Wassertröpfchen der jeweiligen Quick Breaking Emulsionen zu sehen.

Abbildung 1 zeigt eine W/Si-Emulsion mit 0,8 Gew.% Cetyl PEG/PPG-10/1 Dimethicone. Die freigesetzten Wassertröpfen weisen eine Größe von A min: 656,81 µm², Amax: 16166,08 µm² und A mittel: 2753,14 µm² auf; es sind 7,273 Tropfen/mm².

Cetyl PEG/PPG 10/1 Dimethicone ist ein PEG-haltiger Emulgator und es galt eine PEG-freie verbesserte, stabilere und anwendungsfreundlichere Zubereitung mit schnellbrechenden Eigenschaften bereit zu stellen.

Abbildung 2 zeigt eine W/Si-Emulsion mit 0,8 Gew.% Amodimethicone Glycerocarbamat. Die freigesetzten Wassertröpfen weisen eine Größe von A min: 1419,56 µm², Amax: 8284,32 µm² und A mittel: 5850,35 µm² auf; es sind 8,026 Tropfen/mm².

Abbildung 3 zeigt eine W/O-Emulsion mit 1,5 Gew.% Cetyl Diisostearoyl Polyglyceryl-3 Dimer Dilinolate. Die freigesetzten Wassertröpfen weisen eine Größe von A min: 656,81 µm², Amax: 6970,69 µm² und A mittel: 1674,39 µm² auf; es sind 4,783 Tropfen/mm².

Abbildung 4 zeigt eine klassische W/O-Emulsion bei der keine Wassertröpfchen freigesetzt werden. Diese Emulsion enthält zwei Emulgatoren, PEG-40 Sorbitan Perisostearate (1,77%) und Polyglyceryl-3 Diisostearate (1,43%).

Erstaunlich bei den Vergleichen ist auch, dass die Wassertropfengrößenverteilung der erfindungsgemäßen Zubereitungen enger ist als bei den Vergleichszubereitungen.

Die Freisetzung und Ausbildung der Wassertropfen lässt sich erfindungsgemäß über die Menge an erfindungsgemäßen Emulgatoren und/oder den Elektrolyten steuern.

Mit 2 Gew.% Emulgator ist der Effekt weniger ausgeprägt als mit z.B. 0,8 Gew.% Emulgator. Weniger als 0,5 Gew.% Emulgator sollte nicht eingesetzt werden, da die Zubereitungen dann instabil werden können.

Die Emulgatormenge liegt somit bevorzugt zwischen 0,5% und 1,5 Gew.%.

Als Emulgator wird Diisostearoyl Polyglyceryl-3 Dimer Dilinoleat oder Amodimethicone Glycerocarbamat bevorzugt im Bereich zwischen 0,5% und 1,5 Gew.%, gewählt.

Der Anteil an einem oder mehreren Elektrolyten wird bevorzugt im Bereich von 0,1 bis 4 Gew.%, bezogen auf die Gesamtmasse der Zubereitung, gewählt.

Bei höheren Mengen Mg-stearat, z.B. 1%, ist der schnell-brechende Effekt weniger ausgeprägt als mit weniger, z.B. 0,3%. Daher ist der Mg-Stearat-Gehalt bevorzugt zwischen 0,1 Gew.% und 1 Gew.%, bevorzugt zwischen 0,3% und 0,6 Gew.%, bezogen auf die Gesamtmasse der Zubereitung, zu wählen.

Mit höheren Mengen NaCl (1,5%) ist der Effekt stärker als mit weniger, z.B. 0,5%. Bevorzugter NaCl-Anteil liegt daher zwischen 0,1 und 3%, bevorzugt zwischen 0,5% und 1,5%.

Alle Anteilsangaben beziehen sich auf Gewichtsanteile bezogen auf die Gesamtmasse der Zubereitung.

Die erfindungsgemäßen schnell brechenden Zubereitungen sind dadurch charakterisiert, dass sie bei der Applikation auf der Haut Wassertröpfchen freisetzen, was mit einem angenehmen erfrischenden Hautgefühl verbunden ist. Die Haut ist mit sichtbarer Feuchtigkeit versorgt und ein pflegender Rückstand bleibt auf der Haut nach der Produktapplikation. Je größer die Tröpfchen, desto sichtbarer sind sie für den Konsumenten und desto mehr wird der Effekt wahrgenommen.

Die freigesetzten sichtbaren Wassertropfen haben daher eine vorteilhafte Größe von 500 bis 20.000 µm², bevorzugt zwischen 600 und 16.000 µm² ermittelt mit Bildanalyse KS 400 (AxioCam Cc1) auf PMMA-Plättchen, und deren Anzahl beträgt mindestens 3 Tropfen/mm².

Die kosmetischen oder dermatologischen Zubereitungen gemäß der Erfindung können ferner kosmetische Hilfsstoffe und weitere Wirkstoffe enthalten, wie sie üblicherweise in solchen Zubereitungen verwendet werden, z. B. Konservierungsmittel, Konservierungshelfer, Bakterizide, Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, organische Lösungsmittel oder Silikonderivate, Selbstbräuner, Puffer, pH Regulatoren, Pflanzliche Extrakte, Tenside, Treibgase, Puder, Sebumabsorbierende Substanzen, UV-Filter, Wirkstoffe wie zum Beispiel Anti Age, Anti-Cellulite, Anti Akne, Anti-Rosacea, Anti-Neurodermitis, Antioxidantien, Moisturiser, Chelatbildner, Antitraspirantien, Bleich und Färbemittel, sofern der Zusatz die geforderten Eigenschaften hinsichtlich Emulgatorgehalt, geforderter Stabilität, Sensorik und vor allem der Wassertropfenfreisetzung bei der Anwendung nicht behindert.

Bevorzugt umfassen die erfindungsgemäßen Zubereitungen ein oder mehrere kosmetische und/oder dermatologische Wirkstoffe sowie ein oder mehrere Lichtschutzfilter.

Der Wassergehalt der erfindungsgemäßen Zubereitungen liegt vorteilhaft zwischen 70% und 85%, bevorzugt zwischen 72% und 82% und besonders bevorzugt zwischen 76% und 80% jeweils bezogen auf die Gesamtmasse der Zubereitungen.

Die nachfolgenden Beispiele illustrieren die erfindungsgemäßen Zubereitungen. Die Zahlenangaben beziehen sich auf die Gewichtsanteile in Bezug zur Gesamtmasse der Zubereitung, sofern nichts anderes angegeben ist.

### Beispiele

| Inci | Rezeptur C | Rezeptur A | Rezeptur E | Rezeptur G |
|---|---|---|---|---|
| Diisostearoyl Polyglyceryl-3 Dimer Dilinoleate | 0 | 0 | 0 | 1,5 |
| Amodimethicone Glycerocarbamat | 0,8 | 1,5 | 1,5 | 0 |
| Glycerin + Glyceryl Glucoside | 0,1 | 0,1 | 0,1 | 0,1 |
| Dicaprylyl Ether | 5 | 0 | 3,5 | 5 |
| Ethylhexyl Cocoate | 5 | 0 | 3,5 | 5 |
| C13-16 Isoparaffin | 0 | 5 | 0 | 0 |
| Cyclomethicone | 0 | 4 | 0 | 0 |
| Parfum | 0,2 | 0,2 | 0,2 | 0,2 |
| Glycerin | 10 | 10 | 10 | 10 |
| Methylisothiazolinone | 0,09 | 0,09 | 0,09 | 0,09 |
| Phenoxyethanol | 0,6 | 0,6 | 0,6 | 0,6 |
| Magnesium Stearate | 0,3 | 0,3 | 0,3 | 0,3 |
| Sodium Chloride | 1 | 1 | 1 | 1 |
| Aqua | 76,91 | 77,21 | 79,21 | 76,21 |

Die Beispiele zeigen die Verwendungsmöglichkeit von mindestens 0,1 Gew.%, bezogen auf die Gesamtmasse der Zubereitung, an Amodimethicone Glycerocarbamat und/oder Diisostearoyl Polyglyceryl-3 Dimer Dilinoleat in Kombination mit einem oder mehreren Elektrolyten und einem oder mehreren Lipide zur Freisetzung von Wassertropfen aus einer Wasser-in-Öl Emulsionszubereitung auf der Haut. Die freigesetzten Wassertropfen zeigen dabei eine Größe von 500 bis 20.000 µm², bevorzugt zwischen 600 und 16.000 µm² ermittelt mit Bildanalyse KS 400 (AxioCam Cc1) auf PMMA-Plättchen, und deren Anzahl mindestens 3Tropfen/mm² beträgt.

## Patentansprüche

1. Kosmetische oder dermatologische Zubereitung auf Basis einer Wasser-in-Öl Emulsion umfassend jeweils mindestens 0,1 Gew.%, bezogen auf die Gesamtmasse der Zubereitung, eines oder mehrerer W/O-Emulgatoren, eines oder mehrerer Elektrolyte und eines oder mehrerer Lipide **dadurch gekennzeichnet, dass** die Zubereitung Magnesiumstearat umfasst, frei von Polyethylenglykolen und/oder Polyethylenglykolderivaten ist und der oder die W/O-Emulgatoren ausgewählt werden aus der Gruppe Amodimethicone Glycerocarbamat und/oder Diisostearoyl Polyglyceryl-3 Dimer Dilinoleat, wobei der Anteil an dem einen oder den beiden W/O Emulgatoren im Bereich von 0,5 bis 2 Gew.%, bezogen auf die Gesamtmasse der Zubereitung, gewählt wird.

2. Zubereitung nach Anspruch 1 **dadurch gekennzeichnet, dass** bei der Applikation und/oder Verteilen der Zubereitung auf der Haut oder Haar Wassertropfen freigesetzt werden.

3. Zubereitung nach Anspruch 2 **dadurch gekennzeichnet, dass** die freigesetzten Wassertropfen eine Größe von 500 bis 20.000 µm², bevorzugt zwischen 600 und 16.000 µm² ermittelt mit Bildanalyse KS 400 (AxioCam Cc1) auf PMMA-Plättchen, und deren Anzahl mindestens 3 Tropfen/mm² betragen.

4. Zubereitung nach einem der vorstehenden Ansprüche **dadurch gekennzeichnet, dass** der Anteil an einem oder beiden W/O Emulgatoren im Bereich von 0,5 bis 1,5 Gew.%, gewählt wird, bezogen auf die Gesamtmasse der Zubereitung.

5. Zubereitung nach einem der vorstehenden Ansprüche **dadurch gekennzeichnet, dass** nur ein W/O Emulgator enthalten ist, gewählt aus Amodimethicone Glycerocarbamat oder Diisostearoyl Polyglyceryl-3 Dimer Dilinoleat.

6. Zubereitung nach einem der vorstehenden Ansprüche **dadurch gekennzeichnet, dass** der Anteil an einem oder mehreren Elektrolyten im Bereich von 0,1 bis 4 Gew.%, bezogen auf die Gesamtmasse der Zubereitung, gewählt wird.

7. Zubereitung nach einem der vorstehenden Ansprüche **dadurch gekennzeichnet, dass** als Elektrolyt zusätzlich Natriumchlorid gewählt wird.

8. Zubereitung nach einem der vorstehenden Ansprüche **dadurch gekennzeichnet, dass** der Anteil an einem oder mehreren Lipiden im Bereich von 1 bis 20 Gew.%, bezogen auf die Gesamtmasse der Zubereitung, gewählt wird.

9. Zubereitung nach einem der vorstehenden Ansprüche **dadurch gekennzeichnet, dass** als Lipide Dicaprylylether, Dibutyladipat, Isopropylstearat, Octylcocoat, Isopropylpalmitat, Caprylsäure/Caprinsäuretriglycerid, Octyldodecanol einzeln oder als Kombinationen gewählt werden.

10. Zubereitung nach einem der vorstehenden Ansprüche **dadurch gekennzeichnet, dass** der Wassergehalt zwischen 70 Gew.% und 85 Gew.%, bevorzugt zwischen 72 Gew.% und 82 Gew.% und besonders bevorzugt zwischen 76 Gew.% und 80 Gew.% jeweils bezogen auf die Gesamtmasse der Zubereitungen, gewählt wird.

11. Verwendung einer Zubereitung nach einem der vorstehenden Ansprüche zur Freisetzung von Wassertropfen aus der Zubereitung auf der Haut **dadurch gekennzeichnet, dass** die freigesetzten Wassertropfen eine Größe von 500 bis 20.000 µm², bevorzugt zwischen 600 und 16.000 µm² ermittelt mit Bildanalyse KS 400 (AxioCam Cc1) auf PMMA-Plättchen, und deren Anzahl mindestens 3Tropfen/mm² beträgt.

12. Verwendung von 0,5 bis 2 Gew.%, bezogen auf die Gesamtmasse der Zubereitung, an Amodimethicone Glycerocarbamat und/oder Diisostearoyl Polyglyceryl-3 Dimer Dilinoleat in Kombination mit einem oder mehreren Elektrolyten und einem oder mehreren Lipiden zur Freisetzung von Wassertropfen aus einer Wasser-in-Öl Emulsionszubereitung auf der Haut **dadurch gekennzeichnet, dass** die freigesetzten Wassertropfen eine Größe von 500 bis 20.000 µm², bevorzugt zwischen 600 und 16.000 µm² ermittelt mit Bildanalyse KS 400 (AxioCam Cc1) auf PMMA-Plättchen, und deren Anzahl mindestens 3Tropfen/mm² beträgt.

## Claims

1. Cosmetic or dermatological preparation based on a water-in-oil emulsion comprising in each case at least 0.1% by weight, based on the total weight of the preparation, of one or more W/O emulsifiers, of one or more electrolytes and of one or more lipids, **characterized in that** the preparation comprises magnesium stearate, is free of polyethylene glycols and/or polyethylene glycol derivatives and the W/O emulsifier(s) are selected from the group of amodimethicone glycerocarbamate and/or diisostearoyl polyglyceryl-3 dimer dilinoleate, wherein the proportion of one or both W/O emulsifiers is selected in the range of 0.5 to 2% by weight, based on the total mass of the preparation.

2. Preparation according to Claim 1, **characterized in that** water droplets are released when applying and/or spreading the preparation on the skin or hair.

3. Preparation according to Claim 2, **characterized in that** the size of the water droplets released is from 500 to 20 000 µm², preferably between 600 and 16 000 µm², determined using KS 400 image analysis (AxioCam Cc1) on PMMA microplates, and the number thereof is at least 3 droplets/mm².

4. Preparation according to any of the preceding claims, **characterized in that** the proportion of one or both W/O emulsifiers is selected in the range of 0.5 to 1.5% by weight, based on the total mass of the preparation.

5. Preparation according to any of the preceding claims, **characterized in that** only one W/O emulsifier is present, selected from amodimethicone glycerocarbamate or diisostearoyl polyglyceryl-3 dimer dilinoleate.

6. Preparation according to any of the preceding claims, **characterized in that** the proportion of one or more electrolytes is selected in the range of 0.1 to 4% by weight, based on the total mass of the preparation.

7. Preparation according to any of the preceding claims, **characterized in that** sodium chloride is selected as additional electrolyte.

8. Preparation according to any of the preceding claims, **characterized in that** the proportion of one or more lipids is selected in the range of 1 to 20% by weight, based on the total mass of the preparation.

9. Preparation according to any of the preceding claims, **characterized in that** the lipids selected are dicaprylyl ether, dibutyl adipate, isopropyl stearate, octyl cocoate, isopropyl palmitate, caprylic acid/capric acid triglyceride, octyldodecanol, singly or as combinations.

10. Preparation according to any of the preceding claims, **characterized in that** the water content is selected between 70% by weight and 85% by weight, preferably between 72% by weight and 82% by weight and particularly preferably between 76% by weight and 80% by weight, based in each case on the total mass of the preparations.

11. Use of a preparation according to any of the preceding claims for releasing water droplets from the preparation on the skin, **characterized in that** the size of the water droplets released is from 500 to 20 000 µm², preferably between 600 and
16 000 µm², determined using KS 400 image analysis (AxioCam Cc1) on PMMA microplates, and the number thereof is at least 3 droplets/mm².

12. Use of 0.5 to 2% by weight, based on the total mass of the preparation, of amodimethicone glycerocarbamate and/or diisostearoyl polyglyceryl-3 dimer dilinoleate in combination with one or more electrolytes and one or more lipids for releasing water droplets from a water-in-oil emulsion preparation on the skin, **characterized in that** the size of the water droplets released is from 500 to 20 000 µm², preferably between 600 and 16 000 µm², determined using KS 400 image analysis (AxioCam Cc1) on PMMA microplates, and the number thereof is at least 3 droplets/mm².

## Revendications

1. Préparation cosmétique ou dermatologique à base d'une émulsion eau dans huile comprenant à chaque fois au moins 0,1 % en poids, par rapport à la masse totale de la préparation, d'un ou de plusieurs émulsifiants E/H, d'un ou de plusieurs électrolytes et d'un ou de plusieurs lipides, **caractérisée en ce que** la préparation comprend du stéarate de magnésium, est exempte de polyéthylène glycols et/ou de dérivés de polyéthylène glycols, et le ou les émulsifiants E/H sont choisis dans le groupe constitué par le glycérocarbamate d'amodiméthicone et/ou le dilinoléate de diisostéaroyl-polyglycéryl-3 dimère, la proportion du ou des deux émulsifiants E/H étant choisie dans la plage allant de 0,5 à 2 % en poids, par rapport à la masse totale de la préparation.

2. Préparation selon la revendication 1, **caractérisée en ce que** des gouttelettes d'eau sont libérées lors de l'application et/ou de la répartition de la préparation sur la peau ou les cheveux.

3. Préparation selon la revendication 2, **caractérisée en ce que** les gouttelettes d'eau libérées ont une taille de 500 à 20 000 µm², de préférence comprise entre 600 et 16 000 µm², calculée par analyse d'images KS 400 (AxioCam Cc1) sur des plaquettes de PMMA, et leur nombre est d'au moins 3 gouttelettes/mm².

4. Préparation selon l'une quelconque des revendications précédentes, **caractérisée en ce que** la proportion du ou des deux émulsifiants E/H est choisie dans la plage allant de 0,5 à 1,5 % en poids, par rapport à la masse totale de la préparation.

5. Préparation selon l'une quelconque des revendications précédentes, **caractérisée en ce que** seul un émulsifiant E/H est contenu, choisi parmi le glycérocarbamate d'amodiméthicone ou le dilinoléate de diisostéaroyl-polyglycéryl-3 dimère.

6. Préparation selon l'une quelconque des revendications précédentes, **caractérisée en ce que** la proportion du ou des électrolytes est choisie dans la plage allant de 0,1 à 4 % en poids, par rapport à la masse totale de la préparation.

7. Préparation selon l'une quelconque des revendications précédentes, **caractérisée en ce que** du chlorure de sodium est en outre choisi en tant qu'électrolyte.

8. Préparation selon l'une quelconque des revendications précédentes, **caractérisée en ce que** la proportion du ou des lipides est choisie dans la plage allant de 1 à 20 % en poids, par rapport à la masse totale de la préparation.

9. Préparation selon l'une quelconque des revendications précédentes, **caractérisée en ce que** l'éther dicaprylylique, l'adipate de dibutyle, le stéarate d'isopropyle, le cocoate d'octyle, le palmitate d'isopropyle, l'acide caprylique/le triglycéride d'acide caprique, l'octyldodécanol sont choisis individuellement ou en combinaisons en tant que lipides.

10. Préparation selon l'une quelconque des revendications précédentes, **caractérisée en ce que** la teneur en eau est choisie entre 70 % en poids et 85 % en poids, de préférence entre 72 % en poids et 82 % en poids, et de manière particulièrement préférée entre 76 % en poids et 80 % en poids, à chaque fois par rapport à la masse totale des préparations.

11. Utilisation d'une préparation selon l'une quelconque des revendications précédentes pour la libération de gouttelettes d'eau à partir de la préparation sur la peau, **caractérisée en ce que** les gouttelettes d'eau libérées ont une taille de 500 à 20 000 µm², de préférence comprise entre 600 et 16 000 µm², calculée par analyse d'images KS 400 ((AxioCam Cc1) sur des plaquettes de PMMA, et leur nombre est d'au moins 3 gouttelettes/mm².

12. Utilisation de 0,5 à 2 % en poids, par rapport à la masse totale de la préparation, de glycérocarbamate d'amodiméthicone et/ou de dilinoléate de diisostéaroyl-polyglycéryl-3 dimère en combinaison avec un ou plusieurs électrolytes et un ou plusieurs lipides pour la libération de gouttelettes d'eau à partir d'une préparation d'émulsion eau dans huile sur la peau, **caractérisée en ce que** les gouttelettes d'eau libérées ont une taille de 500 à 20 000 µm², de préférence comprise entre 600 et 16 000 µm², calculée par analyse d'images KS 400 (AxioCam Cc1) sur des plaquettes de PMMA, et leur nombre est d'au moins 3 gouttelettes/mm².
